# EUROPEAN PATENT APPLICATION

(11) **EP 2 745 868 A1**
(43) Date of publication of application: **25.06.2014**
(21) Application number: 12199232.5
(22) Date of filing: 21.12.2012
(51) Int. Cl.: A61M 25/00, A61L 29/00, B29C 45/00

(54) **Medical device and method of producing thereof having a tubular substrate and at least partly surface treated access openings**

(71) Applicant: Dentsply IH AB, 431 21 Mölndal (SE)
(72) Inventor: Nyman, Martin, 428 36 Kallered (SE); Utas, Jan, 434 96 Kungsbacka (SE); Dahlberg, Niklas, 412 82 Göteborg (SE); Engström, Axel, 431 21 Mölndal (SE)
(74) Representative: Lind, Urban Arvid Oskar

(57) **Abstract**

A method for producing a medical device and the thereby produced medical device are disclosed. The method comprises the steps of: providing a tubular substrate (3), said tubular substrate enclosing an internal lumen and being provided with at least one recess (51) in the outer surface, wherein said recess does not form a through going hole into the internal lumen; treating the outer surface of said tubular substrate, preferably by means of surface modification and/or by coating; and forming, after said treating of the outer surface, an opening (4) within said at least one recess to form an access opening to said internal lumen.

## Description

### Field of the invention

The present invention relates to a medical device, such as a catheter, comprising a tubular substrate enclosing an internal lumen, and at least one opening providing access to the internal lumen. Further, the invention relates to a method for the production of such a medical device.

### Background of the invention

Tubular substrates having an internal lumen and access openings formed in the wall of the substrate for access to the lumen are used in many types of medical devices, such as in catheters. Such tubular devices are often provided with a surface treatment, such as a surface modification and/or a coating.

However, manufacturing of such medical devices is often complicated and costly. Provision of the access openings prior to surface treatment leads to an undesired surface treatment also of the inside of the device, i.e. within the internal lumen. Provision of the access openings after the surface treatment is also problematic. For example, the surface treatment often make the surface slippery, and difficult to handle. Further, the formation of the access openings often makes the edges of the openings sharp, unsmooth and the like, which may lead to discomfort, pain or even harm to the user.

It is known to coat medical devices, e.g. catheters for introduction into human cavities such as blood vessels, digestive organs and the urinary system, with a hydrophilic coating, at least on the surface of the insertable part which is introduced or comes into contact with mucous membranes, etc., during introduction of the device. An advantage with such a hydrophilic coating is that it becomes extremely slippery when it is swelled with water, preferably immediately before introduction into the human body and thus ensures a substantially painless introduction with a minimum of damage on tissue. Urinary catheters, and in particular urinary catheters for intermittent use, are often provided with such a hydrophilic surface coating. However, if the coating solution enters into the internal lumen, this will be a waste of the coating solution, and may also lead to clogging of the internal lumen, etc. Further, handling of an already coated catheter is difficult, due to the extremely low friction of the surface.

A large number of methods are known for the production of hydrophilic surface coatings. A known hydrophilic coating process is e.g. disclosed in EP 0 093 093, where isocyanate is used to form a polyurea network for connecting hydrophilic PVP to the substrate. Further, EP 0 217 771 describes a method of adding an osmolality increasing compound to such a hydrophilic coating in order to improve the water retention properties and low friction of the coating. Further, WO 98/58989 discloses a hydrophilic coating which is cross-linked by means of irradiation, and incorporating a water soluble osmolality increasing compound therein.

It is further known from e.g. US 2010/0324535 and WO 2010/070048 to form drainage openings into a desired rounded shape afterwards, in a subsequent step. This is made by melting, or otherwise deforming, the edges of the drainage openings. However, such a solution does not address the above-discussed drawbacks, and also makes the production even more cumbersome and costly. Similar methods of forming the edges of a drainage opening in subsequent steps by e.g. grinding is also known from US 2 972 779 and FR 790 544.

Further, it is known to produce drainage openings already during injection molding of the tubular substrate. Such a method is disclosed in US 2012/0150130. The mold is here provided with an inner rod - forming the interior lumen of the catheter - and two "molding members" radially inserted through the mold to be in contact with the unsupported end of the rod. However, this document does also not address the above-discussed problems related to coating and other surface treatments.

Accordingly, there is a need for an improved medical device of the above-discussed type, which has improved access openings, and/or which can be manufactured more cost-efficiently.

### Summary of the invention

It is therefore an object of the present invention to provide a medical device and a method for producing such a medical device, which at least alleviate the above-discussed problems of the previously known solutions.

This object is achieved with a medical device and a method according to the appended claims.

According to a first aspect of the present invention, there is provided a method for producing a medical device, comprising the steps of:
providing a tubular substrate, said tubular substrate enclosing an internal lumen and being provided with at least one recess in the outer surface, wherein said recess does not form a through going hole into the internal lumen;
treating the outer surface of said tubular substrate, preferably by means of surface modification and/or by coating; and
forming, after said treating of the outer surface, an opening within said at least one recess to form an access opening to said internal lumen.

According to another aspect of the present invention, there is provided a medical device comprising a tubular substrate, said tubular substrate enclosing an internal lumen, and being provided with at least one opening providing access to said internal lumen, wherein at least a part of an outer surface of said tubular substrate is provided with a surface treatment, and preferably a surface modification and/or a coating, wherein the internal lumen is free of said surface treatment, and wherein a wall of said at least one opening is at least partly provided with said surface treatment.

By "surface treatment" and "surface treated" is in the present application meant any form of physical or chemical modification of the surface, or addition of material to the surface, such as by means of coating.

The term "tubular substrate" as used herein refers to an object at least part of which forms a tube. The term "tube" here refers to an elongated shaft with a lumen therein. The tube may typically be an elongate hollow cylinder, but may also be a hollow shaft of other cross-sectional shapes. The tube may be opened at both ends, but may also be closed at one or both ends. The tube may also have additional access openings in the side wall, such as drainage openings/eyes, output openings, etc.

The present invention is particularly suited for forming drainage openings, so-called eyes, in the insertable tip portion of a catheter or the like.

The method involves the steps of forming or providing a part of the hole, a recess, from the outside surface of the catheter, but leaving a part of the material towards the interior surface to maintain the surface closed during surface treatment. After surface treatment, e.g. coating, the remaining part of the hole is removed.

Hereby, a number of advantages are obtained. For example, the drainage opening can hereby easily be provided with a suitable geometry, such as rounded edges, thereby making the catheter more comfortable to use, and reducing the risk of pain and harm to the user. Further, the surface treatment, such as a coating process, becomes more efficient. Since the holes are closed during the surface treatment, there is no risk of e.g. coating solution, etching solution or the like, entering into the internal lumen of the tubular substrate. Further, since the hole is already partly made prior to the surface treatment, removal of the remaining part is much easier than the relatively complicated task of e.g. punching holes in an already coated catheter. In addition, the surface treatment will hereby, at least partly, be provided also on the outer surfaces of the opening, making the medical device better aimed at its intended purpose. For example a coated catheter will hereby be to some extent coated also on the walls of the opening(s), thereby making the catheter more comfortable to use, and reducing the risk of pain and harm to the user. Forming of the access opening within the recess is also far easier than forming an opening in the substrate wall where no such recesses are present.

The medical device is preferably a catheter, and more preferably a urinary catheter, and most preferably a urinary catheter for intermittent, short time use. The term "short term use" indicates a use that is limited in time, and in particular limited to a time period of less than 15 minutes, and preferably less than 10 minutes, and most preferably less than 5 minutes.

The recess preferably extends to a depth of at least 10% of a wall thickness of the tubular substrate, and more preferably extends to a depth of at least 50%, and most preferably extends to a depth of at least 75%. Correspondingly, the surface modification and/or coating on the wall of the at least one opening preferably extends over at least 10% of the surface area of the wall, and preferably extends over at least 50%, and most preferably extends over at least 75%. The remaining part of the recess, blocking access to the internal lumen during surface treatment, preferably comprises at least 1% of the initial wall thickness, and more preferably at least 2%, and most preferably at least 5%.

The recess preferably forms an area with reduced wall thickness on the tubular substrate. However, for some embodiments, it is also feasible to provide the recess as an indentation in the tubular wall, wherein the wall thickness may be about the same in the recess as in the rest of the tubular substrate.

The tubular substrate, including the recess(es), may be formed by injection molding. Hereby, the recess can easily be formed with a desired shape, such as rounded external edges and the like. It is also possible to form the recess in various ways which facilitate the subsequent removal of the remaining part of the access openings. For example, the recess may be provided with a very thin wall towards the internal lumen, be provided with local areas of even further reduced thickness, such as tear lines or the like, be provided with outwardly protruding parts functioning as a handle for removing the remaining material, and the like.

Alternatively, the recess(es) may be formed by at least one of milling, punching, drilling and cutting. For example, the recesses may be formed by punching in a lateral direction, perpendicular to the longitudinal direction of the tubular substrate, but displaced from the central axis of the tubular substrate.

According to one embodiment, the material separating the recess from the internal lumen is a different material than the material in the rest of the tubular substrate. This may e.g. be obtained by using two-component injection molding. The material arranged in the recess may hereby be relatively loosely bound to the other material, thereby facilitating subsequent removal. It may also have a lower melting temperature, enabling removal by heating of the medical device, be soluble in a solvent in which the other material is not soluble, or the like. Still further, the material in the recess may be provided with identification means, such as being of an optically identifiable color, containing magnetic material, or the like. Such identification means may be used to identify the remaining material in an automated process.

The surface treatment may involve surface modification. The surface modification preferably involves at least one of plasma treatment, etching and irradiation.

Additionally or alternatively, the surface treatment may comprise coating. The coating process preferably involves at least one of spraying, dipping, incubation and rolling.

In particular, it is preferred that the coating is a hydrophilic coating, comprising a hydrophilic polymer, said hydrophilic polymer exhibiting a low friction when wetted. Such hydrophilic coatings may comprise at least one of: polyvinyl compounds, polylactames, in particular such as polyvinyl pyrrolidones, polysaccharides, in particular heparin, dextran, xanthan gum, derivatised polysaccharides, hydroxy propyl cellulose, methyl cellulose, polyurethanes, polyacrylates, polyhydroxyacrylates, polymethacrylates, polyacrylamides, polyalkylene oxides, in particular polyethylene oxides, polyvinyl alcohols, polyamides, polyacrylic acid, copolymers of the previously mentioned polymers, copolymers of vinyl compounds and acrylates or anhydrides, copolymers of vinylpyrrolidone and hydroxy ethylmethyl acrylate, cationic copolymers of polyvinyl pyrrolidone and copolymer of polymethylvinyl ether and maleinic acid anyhydride. Most preferably, the hydrophilic coating comprises polyvinyl pyrrolidone.

The hydrophilic coating preferably forms a polyurea network, and most preferably the polyurea network is arranged to form a covalent bond to active hydrogen groups in the substrate. Alternatively, the hydrophilic coating may form an ester bond or an epoxy bond to active hydrogen groups in the substrate.

According to one embodiment, coating of the substrate material may be made by a process comprising the steps of: applying sequentially to the surface of the substrate first a solution comprising between 0.05 to 40% (weight to volume) of an isocyanate compound and thereafter a solution containing between 0.5 and 50% (weight to volume) of polyvinylpyrrolidone and curing at an elevated temperature.

However, other hydrophilic coatings are also feasible, such as a coating comprising hydrophilic polymers cross-linked directly to the substrate. The crosslinking may be effected by means of irradiation, e.g. by electron beams or UV light.

The tubular substrate may be formed of a large variety of different substrate materials. However, preferably the substrate is made of a polymer material. The substrate may e.g. comprise at least one of: polyurethanes, latex rubbers, silicon rubbers, other rubbers, polyvinylchloride (PVC), other vinyl polymers, polyesters, polyacrylates, polyamides, polyolefines, thermoplastic elastomers, styrene block copolymers (SBS), or polyether block amid (PEBA).

The access opening(s) preferably comprises an internal edge abutting the internal lumen and an external edge abutting the outer surface, wherein the external edge of the opening is a substantially rounded edge. However, various slanted dispositions, etc. are also feasible. Hereby, a relatively smooth entrance to the access openings are provided. By providing the outer edge with a rounded or slanted edge, it may be possible to minimize the discomfort or the trauma to the urethra during insertion when used in urinary catheters, and similar advantages are obtained for other applications. The edges of the access opening/drainage eyes may e.g. be shaped in the way discussed in US 2010/0324535 and WO 2010/070048, both said documents hereby incorporated by reference.

After the surface treatment, the remaining part of the recess may be removed in various ways. The opening may e.g. be formed by punching, or by drilling, ripping, cutting, milling, grinding or the like. Laser cutting, water cutting, die cutting and the like may also be used. Blowing, e.g. from within the internal lumen, may also be used. Alternatively, the remaining part may be removed by pulling, e.g. by applying a force to a handle formed in the remaining part, using a suction cup or the like, by pushing, e.g. by application of an overpressure within the internal lumen, or the like. As discussed above, the remaining material may also be removed by melting, dissolving or the like. This is particularly useful if different materials are used in the recess and in the rest of the substrate. However, even if the same material is used, e.g. dissolving may be used, in particular if the remaining material in the recess is very thin.

The remaining material is preferably removed during manufacturing. However, it is also possible to remove the remaining material subsequently, during storage or during preparation for use of the medical device. For example, a material being dissolvable in water, saline, urine or the like, may be removed by storing the medical device in a storage solution. For example, it is known to store hydrophilic urinary catheters in a wetting liquid to maintain the catheter in a ready-to-use state. For such products, the remaining material may be arranged to dissolve during storage.

It is also possible to provide the remaining material in a form that dissolves in a wetting fluid during wetting/activation of the medical device prior to use. Still further, it is also possible to provide a remaining material which dissolves when brought into contact with bodily fluids during use, such as by urine. Examples of such materials are monosaccharide, disaccharide, oligosaccharide and polysaccharide. For example, the tube material may primarily comprise water, at least one of sugar and starch and gelatin. Tube materials comprising these ingredients may form degradable materials that may be totally dissolved if maintained in water. Examples of such materials are known from the previous application with application number EP 09171080 by the same applicant, which is hereby incorporated by reference.

The production method and medical device is particularly useful for catheters, and in particular urinary catheters. However, it is also useful for many other types of medical devices. Accordingly, the method and medical device according to the present invention is not limited to urinary catheters. Examples of such other medical devices for which the present invention is useful are vascular catheters and other types of catheters, endo and laryngoscopes, tubes for feeding, or drainage or endotracheal use and devices for circulatory assistance.

These and other aspects of the invention will be apparent from and elucidated with reference to the embodiments described hereinafter.

### Brief description of the drawings

By way of example, embodiments of the invention will now be described with reference to the accompanying drawings in which:
Fig. 1 illustrates a perspective view of an embodiment of a catheter according to the invention;
Fig. 2 illustrates a cross-sectional view in the radial direction of the tubular susbtrate a first punching step, forming part of a first embodiment of a method in accordance with the invention;
Fig. 3 illustrates a cross-sectional view in the radial direction of the tubular substrate of a second punching step, forming part of a first embodiment of a method in accordance with the invention;
Fig. 4 illustrates a schematic cross-sectional view of a mold for use in another embodiment of the method according to the invention; and
Figs. 5A-5D illustrate cross-sectional views of in the longitudinal direction of the tubular susbtrate of wall portion including recesses of various shapes in accordance with various embodiments of the present invention.

### Description of preferred embodiments

In the following detailed description preferred embodiments of the invention will be described. However, it is to be understood that features of the different embodiments are exchangeable between the embodiments and may be combined in different ways, unless anything else is specifically indicated. The medical devices may be used for many different purposes, and for insertion into various types of body-cavities. However, the following discussion is in particular concerned with the preferred field of use, urinary catheters, even though the invention is not limited to this particular type of catheters or even this particular type of medical device. It is to be appreciated by those skilled in the art that the inventive concept is not limited to any certain type of devices, but could be used in different types of medical devices.

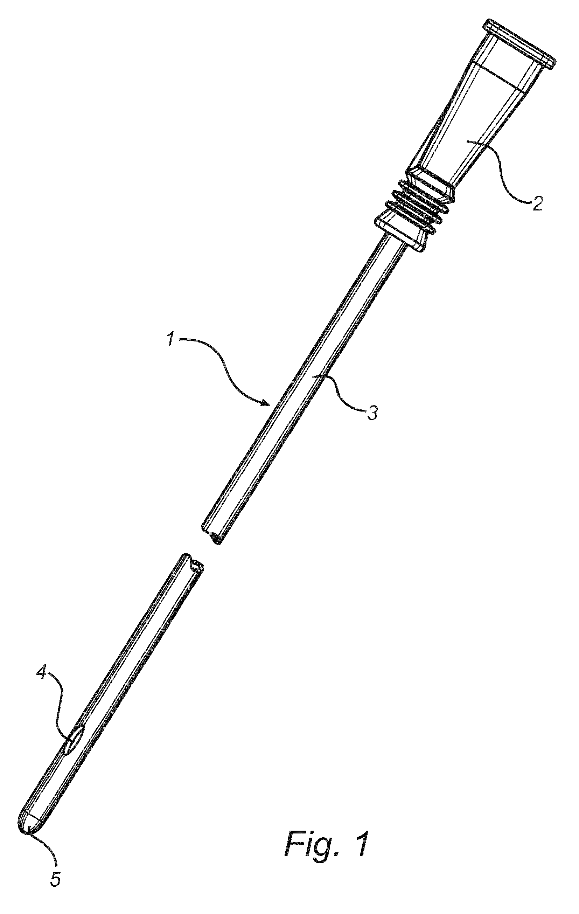

A catheter 1 as illustrated in Fig. 1, comprises a flared rearward portion 2 and an elongate shaft or tube 3 projecting forwardly from the rearward portion 2. An open-ended internal lumen (not shown) extends from the rear end of the rearward portion 2 to a drainage aperture 4 in a rounded tip 5 of the elongate tube 3. The rearward portion 2 may function as a connector of the catheter 1, being connectable to other devices, such as a urine collection bag, a drainage tube or the like.

At least a part of the elongate tube 3 forms an insertable length to be inserted through a body opening of the user, such as the urethra in case of a urinary catheter. By insertable length is normally meant that length of the elongate tube 2 which is coated with a hydrophilic material, for example PVP, and which is insertable into the urethra of the patient. Typically, this will be 80-140 mm for a female patient and 200-350 mm for a male patient.

The elongate shaft/tube of the catheter is made of a substrate material. The substrates may be made from any polymer material, which are well-known in the technical field and to which the said hydrophilic polymers adhere, such as polyurethanes, latex rubbers, other rubbers, polyvinylchloride, polyether block amide (PEBA), other vinyl polymers, polyesters and polyacrylates. However, preferably the substrate is made of a polymer blend comprising a polyolefin and a composition having molecules with active hydrogen groups, and preferably a composition having molecules with active hydrogen groups. The polyolefin can comprise at least one polymer selected from the group: polyethene, polypropene, and styrene block copolymer (SBS). Other thermoplastic elastomers may also be used. The composition having molecules with active hydrogen groups can be a polymer having active hydrogen groups bound to the polymer via nitrogen, such as polyamide or polyurethane, or via oxygen, such as polyvinyl alcohol and poly acrylic acid.

The catheter is further surface treated, by means of surface modifications and/or by coating, as have been discussed in the foregoing. In particular, for catheters, it is preferred to coat the outer surface, at least of the insertable part, with a hydrophilic coating. Many different types of well-known hydrophilic surfaces can be used. For example, the catheter may be provided with a hydrophilic coating wherein the hydrophilic polymer coating comprises material selected from polyvinyl compounds, polysaccharides, polyurethanes, polyacrylates or copolymers of vinyl compounds and acrylates or anhydrides, especially polyethyleneoxide, polyvinylpyrrolidone, heparin, dextran, xanthan gum, polyvinyl alcohol, hydroxy propyl cellulose, methyl cellulose, copolymer of vinylpyrrolidone and hydroxy ethylmethyl acrylate or copolymer of polymethylvinyl ether and maleinic acid anyhydride. The preferred hydrophilic polymer is polyvinylpyrrolidone. A detailed description of a preferred coating method is provided below.

Upon use, the catheter is then wetted by a wetting fluid, whereby the hydrophilic surface becomes slippery and easy to insert into e.g. the urethra of the patient, i.e. to provide a low-friction character of the surface. The wetting fluid is preferably a water-based liquid, i.e. using water as a solvent.

Formation of the drainage openings is made in a two-step procedure. First, one or several recess(es) is/are formed in the tubular substrate material. These recesses do not extend all the way through the substrate wall, and does, thus, not form a through going hole into the internal lumen. These recesses are formed prior to the surface treatment, such as the coating step. After the surface treatment, the openings are completed by removal of the remaining material in the bottom of the recesses. Opening(s) within the at least one recess are thereby formed, creating access opening(s) to the internal lumen.

This two-step procedure may be performed in various ways. In the following, one method involving two step punching and another method involving injection molding will be explained in more detail. However, it is to be appreciated by the skilled reader, that many alternative methods are also feasible, as has been discussed in more detail in the foregoing.

In the first exemplary two-step procedure for forming the openings, a recess is first cut in the substrate wall, to form a recess but not entirely cut through the substrate wall. Such partial cutting may e.g. be obtained by means of punching in a lateral direction, perpendicular to the axial direction of the substrate, and in a direction which is displaced in relation to the center of the substrate. If e.g. a circular punch or cutting edge is used, this will result in a recess having somewhat rounded edges.

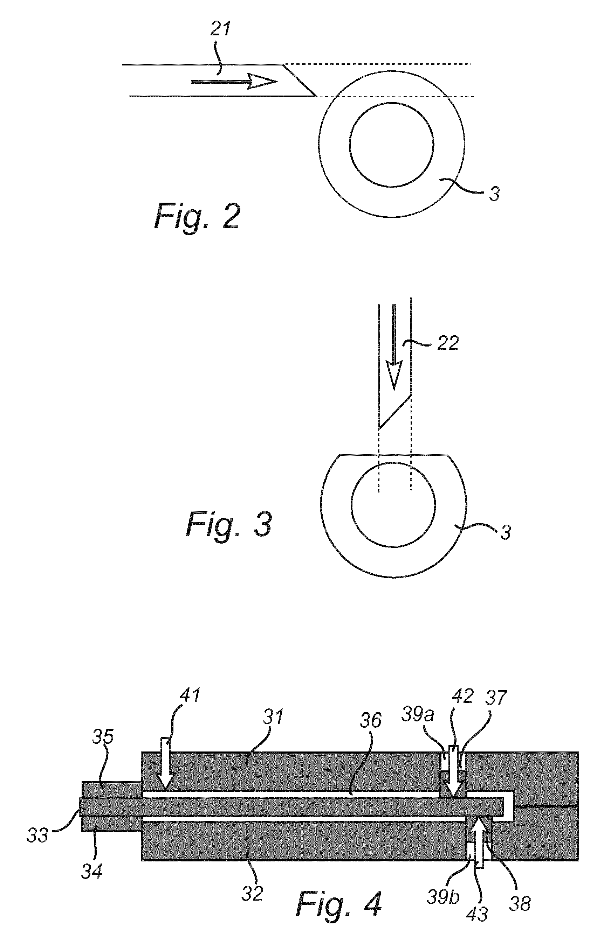

Such a partial punching is illustrated schematically in the cross-sectional view of Fig. 2. To obtain the precision necessary, the substrate is preferably held in a fixture, manufacturing jig or the like, in which the punch or cutter 21 is guided along the desired path. Such manufacturing devices are per se known in the art, e.g. from EP 1 106 200 and US 2006/0027063, both said documents hereby being incorporated by reference.

However, the recess may also be formed by drilling, grinding and the like. For example, the grinding method disclosed in FR 790 544 or the drilling method disclosed in US 4 259 276 may be used for forming the recesses. Both said documents are hereby also incorporated by reference.

When the recesses have been provided, the substrate is subject to surface treatment. Since the recess still forms a closure relative to the internal lumen, the surface treatment will be applied to the outer surface of the substrate, including the walls and bottom of the recess(es), but will not be applied to the interior side of the tubular substrate.

After the completion of the surface treatment, the hole or opening is finalized. This may e.g. be obtained by a second punching or cutting step. This second cutting or punching is preferably made by moving a second punch/cutter 22 in a direction perpendicular to the axial direction of the substrate, and also perpendicular to the first punching direction, and also in line with the center axis of the substrate. The punching/cutting is made in the thin bottom of the recess(es). Such a second punching or cutting action is illustrated schematically in Fig. 3. Hereby, a through going hole or opening is formed within the recess. Further, the slanted or rounded side walls of the recess preferably remains uncut, and thereby form rounded or slanted upper edges of the access opening, whereas the lowermost part of the access opening has straight edges, due to the second punching/cutting.

Thus, by the above-discussed two-step punching/cutting method, an access opening is provided which may easily be provided with a desired upper design, such as having slanted or rounded edges, which provides the surface treatment, such as a coating, also on the upper part of the access hole, which avoids surface treatment on the interior, and which is cost-efficient and quick.

In an alternative exemplary method, the recesses are formed during injection molding of the catheter. Such injection molding may e.g. be performed generally in the way disclosed of US 2012/0150130, said document hereby being incorporated in its entirety by reference.

Here, the tubular substrate, e.g. a catheter, is injection molded, and so that one or several recesses are formed on the side walls of the catheter tube. The molding is preferably made in a mold having an elongated cavity having a longitudinal axis and having a cylindrical shape defining an external surface of the catheter, and with a core pin to be provided inside the cavity in the longitudinal axis of elongated cavity, where the core pin is in the form of an inner lumen of the catheter. The core pin may have a distal end that is fixed in place and a proximal free end. The mold may be prepared for molding by arranging the free ends of two molding members laterally to be almost in contact with the core pin, injecting a liquid catheter material into the mold, letting the liquid material solidify, and withdrawing the two molding members from the core pin in a radial direction away from the core pin, and removing the catheter assembly from the mold.

The terms proximal and distal directions may be seen as in view of the user during insertion, i.e. the proximal end is the end closest to the user and the distal end faces away from the user.

For injection molding, a liquid catheter material to be injected is preferably used, such as a thermoplastic material. Suitable thermoplastic materials may be materials such as polyurethane, polyvinyl chloride, polyethylene and other thermoformable materials. The use of thermoplastic materials means that the construction or the shape of the catheter may be partly or fully provided by treating the catheter or the catheter material with heat, such as melting or by solidifying the material by cooling.

Fig. 4 shows a schematic cross-sectional view of a catheter mold useable in such a method. A first mold half 31 and a second mold half 32 defines a mold cavity 36 when the first mold half 31 and the second mold half 32 are joined along the longitudinal axis of the mold cavity. During the injection molding process, the mold halves 31, 32 are joined so that the inner surfaces defines the external surface of a catheter tube. Subsequent to the injection molding of the liquid catheter material and upon solidification of the material, the halves 31, 32 may be separated to expose the injection molded catheter assembly.

A core pin 33 is provided inside the cavity 36 where the core pin 33 defines the inner lumen of the catheter and the core pin 33 has a distal fixed end and a proximal free end. The distal fixed end of the core pin 33 is fixed in place during the injection molding process, using a fixing apparatus 34, 35. After solidification of the catheter material, the core pin 33 is withdrawn from the cavity 36 and from the inner lumen of the finished catheter tube.

At the proximal end of the core pin 33, a first 37 and second 38 molding member which may be moved close to the core pin 33 during the injection molding process are provided. The first and second molding member 37, 38 may be displaced along a radial axis to the core pin 33, so that during the injection molding process the molding members are displaced to be close to the core pin 33. Hereby, recesses are formed in the injection molded catheter.

The liquid catheter material used to mold the catheter is injected using an injection sprout 41 which fills up the cavity 36 under high pressure, so that all the volume of the cavity 36 is filled up with liquid catheter material. Subsequent to the solidification of the catheter material, the molding members 37, 38 may be withdrawn from the cavity 36 along their tracks 39a, 39b, and subsequently, the first and the second molding halves 31, 32 may be separated. Upon separation of the first and the second molding halves 31 32, the core pin 33 may be withdrawn from the molded material and the may be released from the mold halves.

However, the mold may alternatively be a single piece mold having an elongated cavity, where the elongated cavity may be in the form of the external surface of the catheter assembly and the catheter assembly may be removed from the mold by a withdrawal along the longitudinal axis of the catheter assembly.

Further, the mold may comprise other features, such as a part forming a connector at the distal end of the catheter tube. Alternatively, a connector and other elements of the catheter may be added to the catheter assembly subsequent to the molding.

The molding members may be provided with various shapes, thereby forming the recesses to any shape desired. Hereby, the access openings/drainage eyes of the catheter may e.g. have an outer edge that is rounded. For example, the first and the second molding members may have such a shape that the outer edge of the at least two drainage holes may be rounded during the injection molding. Thus, the provision of the drainage eyes during the injection molding using the first and the second molding members may be modified so that the form of an outer periphery of the molding members corresponds to a rounded outer edge of the drainage eye. Thus, the molding members may for example be a concave surface area, a linear surface area or any surface area that may form a rounded edge.

As discussed in the foregoing, the recesses may be formed of the same material as the rest of the tubular substrate. However, it is also possible to use a different material in the recesses, in order e.g. to allow the bottom material in the recesses to be removed by means of melting, dissolving or the like. If such a different material is to be used, this may be provided by two component molding. For example, the mold of Fig. 4 may be used for such two component molding. Hereby, the molding members 37, 38 may initially be brought into contact with the core pin 33. This may also have the advantageous effect of stabilizing the core pin during the first molding step. After having introduced the liquid catheter material, and after having allowed this material to be solidified, the molding members 37, 38 may be displaced radially outwardly a short distance. The gap hereby formed between the molding members 37, 38 may then be filled with a different material, injected through injection sprouts 42, 43.

After formation of the injection molded catheter, including the recesses, the substrate is subject to surface treatment. Since the recess still forms a closure relative to the internal lumen, the surface treatment will be applied to the outer surface of the substrate, including the walls and bottom of the recess(es), but will not be applied to the interior side of the tubular substrate.

After the completion of the surface treatment, the hole or opening is finalized. This may e.g. be obtained by a punching or cutting step, as disclosed in relation to the previous embodiment. Alternatively, the bottom material in the recess(es) may be removed by e.g. melting or dissolving, as has been discussed in the foregoing. Hereby, a through going hole or opening is formed within the recess. As in the previous embodiment, the slanted or rounded side walls of the recess preferably remains, and thereby form rounded or slanted upper edges of the access opening, whereas the lowermost part of the access opening has straight edges, due to the second punching/cutting.

Thus, also by the above-discussed method, an access opening is provided which may easily be provided with a desired upper design, such as having slanted or rounded edges, which provides the surface treatment, such as a coating, also on the upper part of the access hole, which avoids surface treatment on the interior, and which is cost-efficient and quick.

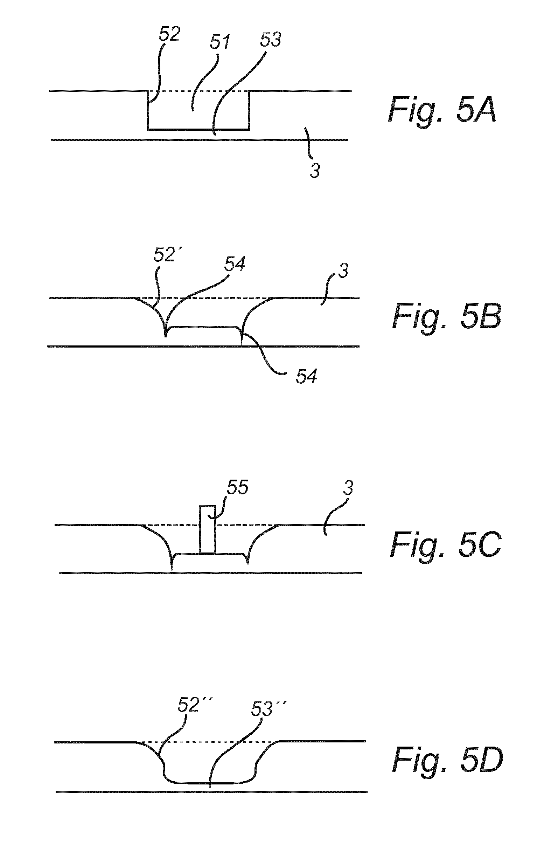

By the above-discussed or other methods, recesses of various shapes may be formed. Some examples of such recesses are shown in Fig. 5A-5D.

As shown in Fig. 5A, the recess 51 may have straight walls 52, being essentially perpendicular to the longitudinal direction of the tubular substrate. A thin remaining bottom 53 is formed, which may be removed after surface treatment.

However, preferably the upper part of the recess is formed with slanted or rounded edges 52', as is schematically shown in Fig. 5B. Further, as is also seen in this figure, tear lines 54 or the like may be formed at the bottom, to facilitate removal of the bottom part.

In Fig. 5C, a similar recess as is shown in Fig. 5B is illustrated. However, here the bottom is provided with a radially upwardly protruding handle 55, which may be used for e.g. tearing off the remaining bottom after surface treatment.

In Fig. 5D, an alternative recess configuration with rounded edges 52" is shown, also exhibiting a thin bottom 53", but without any tear lines.

As would be apparent from the foregoing, many alternative recess configurations are feasible as well.

The person skilled in the art realizes that the present invention by no means is limited to the preferred embodiments described above. On the contrary, many modifications and variations are possible within the scope of the appended claims. For instance, alternative methods for forming recesses, and for removing a remaining bottom material are feasible. Further, the tubular substrate may be used in many medical applications. Still further, the access openings/drainage eyes may be provided in many various shapes. Such and other modifications should be construed to fall within the scope of the appended claims.

## Claims

1. A method for producing a medical device, comprising the steps of:
providing a tubular substrate, said tubular substrate enclosing an internal lumen and being provided with at least one recess in the outer surface, wherein said recess does not form a through going hole into the internal lumen;
treating the outer surface of said tubular substrate, preferably by means of surface modification and/or by coating; and
forming, after said treating of the outer surface, an opening within said at least one recess to form an access opening to said internal lumen.

2. The method of claim 1, wherein the medical device is a catheter, and preferably a urinary catheter, and most preferably a urinary catheter for intermittent use.

3. The method of claim 1 or 2, wherein the recess extends to a depth of at least 10% of a wall thickness of the tubular substrate, and preferably extends to a depth of at least 50%, and most preferably extends to a depth of at least 75%.

4. The method of any one of the preceding claims, wherein the tubular substrate, including said recess(es) is formed by injection molding.

5. The method of any one of the claims 1-3, wherein the recess(es) is formed by at least one of milling, punching, drilling and cutting.

6. The method of any one of the preceding claims, wherein a material separating the recess from the internal lumen is a different material than the material in the rest of the tubular substrate.

7. The method of any one of the preceding claims, wherein the step of treating the outer surface comprises coating, said coating involving at least one of spraying, dipping, incubation and rolling.

8. The method of any one of the preceding claims, wherein the step of treating the outer surface comprises surface modification, said surface modification involving at least one of plasma treatment, etching and irradiation.

9. A medical device comprising a tubular substrate, said tubular substrate enclosing an internal lumen, and being provided with at least one opening providing access to said internal lumen, wherein at least a part of an outer surface of said tubular substrate is provided with a surface treatment, and preferably a surface modification and/or a coating, wherein the internal lumen is free of said surface treatment, and wherein a wall of said at least one opening is at least partly provided with said surface treatment.

10. The medical device of claim 9, wherein the medical device is a catheter, and preferably a urinary catheter, and most preferably a urinary catheter for intermittent use.

11. The medical device of claim 9 or 10, wherein said surface modification and/or coating on the wall of said at least one opening extends over at least 10% of the surface area of said wall, and preferably extends over at least 50%, and most preferably extends over at least 75%.

12. The medical device of any one of the claims 9-11, wherein the tubular substrate is made of a polymeric material.

13. The medical device of any one of the claims 9-12, wherein said surface treatment is a coating comprising a hydrophilic polymer, said hydrophilic polymer exhibiting a low friction when wetted.

14. The medical device of claim 13, wherein the hydrophilic polymer is at least one of: polyvinyl compounds, polylactames, in particular such as polyvinyl pyrrolidones, polysaccharides, in particular heparin, dextran, xanthan gum, derivatised polysaccharides, hydroxy propyl cellulose, methyl cellulose, polyurethanes, polyacrylates, polyhydroxyacrylates, polymethacrylates, polyacrylamides, polyalkylene oxides, in particular polyethylene oxides, polyvinyl alcohols, polyamides, polyacrylic acid, copolymers of the previously mentioned polymers, copolymers of vinyl compounds and acrylates or anhydrides, copolymers of vinylpyrrolidone and hydroxy ethylmethyl acrylate, cationic copolymers of polyvinyl pyrrolidone and copolymer of polymethylvinyl ether and maleinic acid anyhydride.

15. The medical device of any one of the claims 9-14, wherein the hydrophilic polymer is polyvinyl pyrrolidone.

16. The medical device of any one of the claims 9-15, wherein said at least one opening comprises an internal edge abutting the internal lumen and an external edge abutting the outer surface, wherein the external edge of the opening is a substantially rounded edge.
